# EUROPÄISCHE PATENTSCHRIFT

(11) **EP 0 690 047 B1**
(45) Veröffentlichungstag und Bekanntmachung des Hinweises auf die Patenterteilung: **20.05.1998**
(21) Anmeldenummer: 95109168.5
(22) Anmeldetag: 14.06.1995
(51) Int. Cl.: C07C 255/55, C07C 69/90, C09K 19/20

(54) **Substituierte Phenyl 4-[(E)-alk-2-enoyloxy] benzoate**
Substituted phenyl 4-[(E)-alk-2-enoyloxy] benzoates
Phényl 4-[(E)-alk-2-énoyloxy] benzoates substitués

(30) Priorität: 29.06.1994 CH 2067/94
(43) Veröffentlichungstag der Anmeldung: 03.01.1996
(73) Patentinhaber: MERCK PATENT GmbH, 64293 Darmstadt (DE)
(72) Erfinder: Kelly, Stephen, CH-4313 Möhlin (CH); Schadt, Martin, CH-4411 Seltisberg (CH)

(56) Entgegenhaltungen:
- EP-A- 0 034 350
- EP-A- 0 241 338
- EP-A- 0 445 628
- FR-A- 2 246 542
- HELVETICA CHIMICA ACTA, Bd.67, 1984 Seiten 1572 - 1579 S.M. KELLY

## Beschreibung

Die vorliegende Erfindung betrifft neue Verbindungen mit einem 4-[(E)-Alk-2-enoyloxy]benzoatrest, flüssigkristalline Gemische, die solche Verbindungen enthalten, sowie die Verwendung solcher Verbindungen bzw. Gemische für elektro-optische Anzeigevorrichtungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gas/Wirt-Zellen, TN-Zellen ("twisted nematic") mit verdrillt nematischer Struktur, STN-Zellen ("super twisted nematic"), SBE-Zellen ("super birefringence effect") und OMI-Zellen ("optical mode interference"). Die gebräuchlichsten Anzeigevorrichtungen beruhen auf dem Schadt-Helferich-Effekt und besitzen eine verdrillt nematische Struktur.

Die Flüssigkristallmaterialien sollten eine gute chemische und thermische Stabilität haben und ausserdem gegenüber elektrischen Feldern und elektromagnetischen Strahlungen stabil sein. Sie sollten bei betriebsüblichen Temperaturen eine geeignete Mesophase besitzen, beispielsweise eine nematische, cholesterische oder getiltete smektische Phase. Die Flüssigkristallmaterialien sollten ferner niedere Viskosität aufweisen, in den Zellen kurze Ansprechzeiten, tiefe Schwellenspannungen (V₁₀) und einen hohen Kontrast ergeben.

Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit niedriger dielektrischer Anisotropie besteht jedoch in letzter Zeit ein vermehrtes Interesse an Materialien mit sehr hoher dielektrischer Anisotropie und gleichzeitig einem hohen Klärpunkt, insbesondere für TN-Zellen mit niedriger Schwellenspannung, z.B. bei TN-Anwendungen für sogenannte Outdoor-Anwendungen.

Da Flüssigkristalle zwecks Optimierung der Eigenschalten in der Regel als Mischungen mehrerer Komponenten verwendet werden, ist es wichtig, dass die Komponenten untereinander gut mischbar sind. Komponenten, welche in Mischungen schon in kleinen Konzentrationen eine starke Herabsetzung der Schwellenspannung bewirken, haben in der Regel den Nachteil, dass sie den Klärpunkt stark herabsetzen und die Viskosität signifikant erhöhen. Es stellte sich daher die Aufgabe nach Verbindungen zu suchen, deren Einfluss auf die Schwellenspannung (V₁₀) genügend gross ist, dass sie in Konzentrationen eingesetzt werden kann, bei welchen weder eine unerwünschte Klärpunktsherabsetzung noch eine unerwünscht hohe Viskosität auftritt.

Derartige Verbindungen werden nun durch die vorliegende Erfindung zur Verfügung gestellt. Gegenstand der vorliegenden Erfindung sind somit Verbindungen der allgemeinen Formel 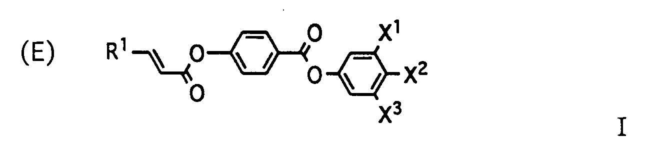 worin
- R¹: Alkyl mit 1 bis 12 Kohlenstoffatomen;
- X¹, X³: unabhängig voneinander Halogen oder Wasserstoff; und
- X²: Halogen oder Cyano bezeichnen.

Es wurde gefunden, dass die Einführung einer (E)-Alk-2-enoylgruppe in die bekannten 4-Cyano-3-fluorophenyl 4-alkylbenzoate (Helv. Chim. Acta, 67, 1572, 1984) die Tendenz zur Ausbildung von Flüssigkristallphasen, vor allem die Tendenz zur Ausbildung einer nematischen Phase, sowie zu hoher dielektrischer Anisotropie günstig beeinflusst. Diese Ester führen in nematischen Mischungen zu hohen Klärpunkten und zu überraschend kurzen Schaltzeiten. Die dielektrische Anisotropie (Δε) und die optische Anisotropie (Δn) sind überraschend gross. Die Schwellenspannung (V₁₀) wird durch die Zugabe der erfindungsgemässen Verbindungen zu Flüssigkristallmischungen sehr stark herabgesetzt.

Bevorzugte Reste R¹ sind Alkylreste mit 1 bis 12 Kohlenstoffatomen, wie beispielsweise Methyl, Ethyl, Propyl, Butyl, Pentyl, Hexyl, Heptyl, Octyl oder Nonyl und dergleichen. Ganz besonders bevorzugt sind Alkylreste mit 1 bis 7, insbesondere mit 1 bis 3, Kohlenstoffatomen.

Der Ausdruck "Halogen" bezeichnet Fluor, Chlor, Brom und Jod, insbesondere jedoch Chlor und Fluor.

Bevorzugte Verbindungen der Formel I sind diejenigen Verbindungen, worin X¹ Fluor, X² Chlor, Fluor oder Cyano und X³ Wasserstoff oder Fluor bedeuten, insbesondere die Verbindungen der allgemeinen Formeln 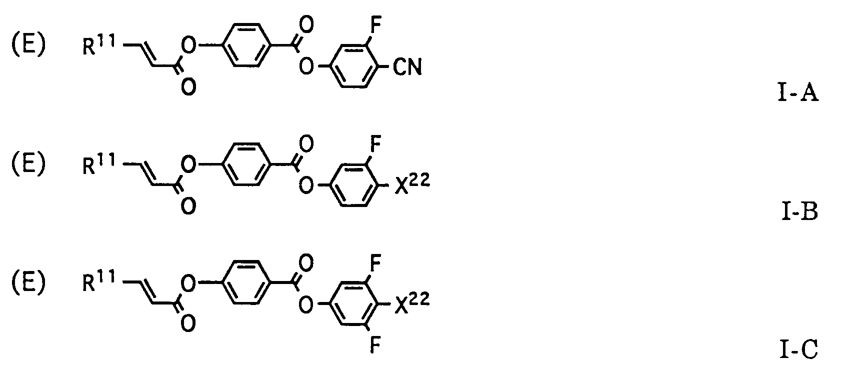 worin
- R¹¹: Alkyl mit 1 bis 7 Kohlenstoffatomen; und
- X²²: Fluor oder Chlor bedeutet.

Ein bevorzugter Aspekt der Erfindung betrifft Verbindungen der Formeln I, I-A, I-B und I-C, worin R¹ bzw. R¹¹ Alkyl mit 1 bis 3 Kohlenstoffatomen bedeutet. Ein ganz besonders bevorzugter Aspekt dieser Erfindung betrifft somit die Verbindungen der Formeln 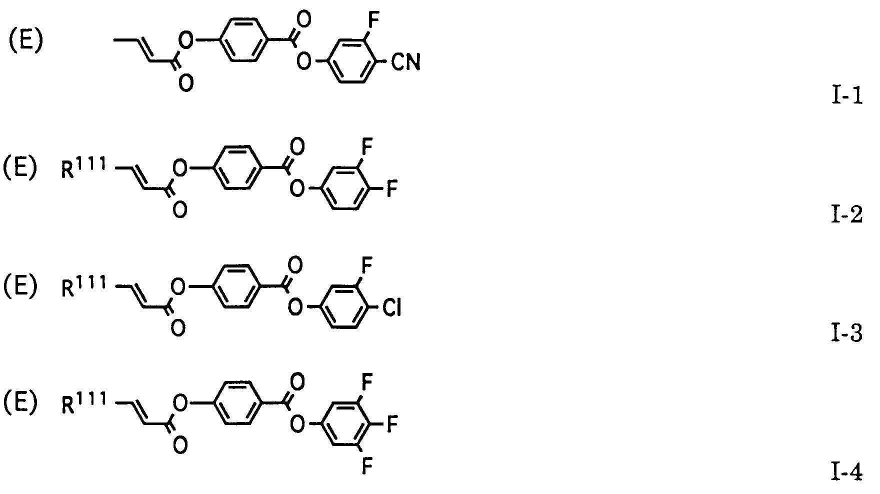 worin R¹¹¹ Alkyl mit 1 bis 3 Kohlenstoffatomen bedeutet.

Die Verbindungen zeichnen sich durch eine überraschend breite nematische Mesophase und durch eine sehr hohe dielektrische Anisotropie (Δε) aus.

Die Verbindungen der Formel I, insbesondere diejenigen der Formel I-A und ganz besonders diejenige der Formel I-1, eignen sich dank ihrer niedrigen Schwellenspannung und ihrem hohen Klärpunkt ganz besonders für die Anwendung in Flüssigkristallmaterialien, welche für TN-Zellen für Outdoor-Anwendungen verwendet werden. Die vorliegende Erfindung stellt somit hervorragende neue Komponenten zur Optimierung und Modifizierung von Flüssigkristallmaterialien zur Verfügung.

Die erfindungsgemässen Verbindungen der allgemeinen Formel I können auf an sich bekannte Weise, beispielsweise nach der im nachstehenden Schema sowie in den Beispielen illustrierten Methode, hergestellt werden. 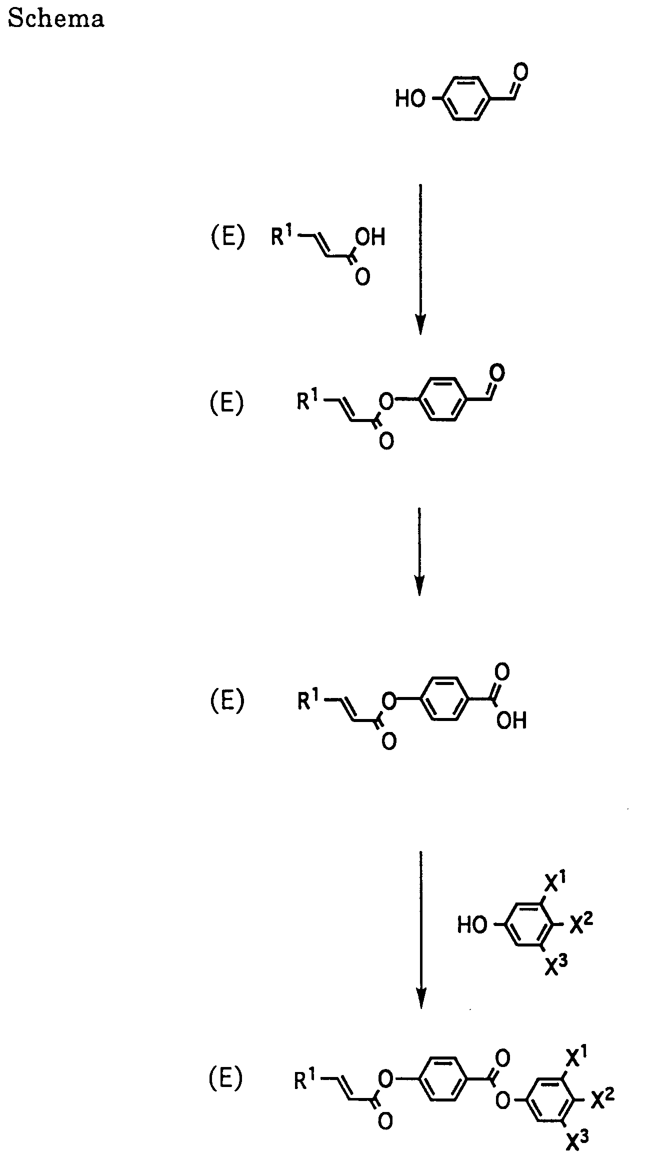 worin R¹, X¹, X², und X³ die in Formel I definierten Bedeutungen haben.

Die erfindungsgemässen Verbindungen der allgemeinen Formel I sind synthetisch leicht zugänglich und können in an sich bekannter Weise aus Phenolen und 4-[(E)-Alk-2-enoyloxy]benzosäuren hergestellt werden. Die Veresterung kann beispielsweise in Gegenwart von N,N'-Dicyclohexylcarbodiimid und 4-(Dimethylamino)pyridin in Dichlormethan oder einem anderen geeigneten Lösungsmittel, wie z.B. Chloroform, erfolgen. Die 4-[(E)-Alk-2-enoyloxy]benzosäuren können aus dem Veresterungsprodukt von 4-Hydroxybenzaldehyd und (E)-Alk-2-ensäuren durch Oxidation mit Jones' Reagenz hergestellt werden. Die Ausgangsmaterialien sind bekannt und z.T. im Handel erhältlich.

Die Verbindungen der Formel I können in Form von Gemischen untereinander und/oder mit anderen Flüssigkristallkomponenten verwendet werden.

Die Erfindung betrifft daher ebenfalls flüssigkristalline Gemische mit mindestens 2 Komponenten, welche dadurch gekennzeichnet sind, dass mindestens eine Komponente eine Verbindung der Formel I ist. Eine zweite und gegebenenfalls weitere Komponenten können weitere Verbindungen der allgemeinen Formel I oder andere geeignete Flüssigkristallkomponenten sein. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-70 Gew.-% betragen. Im allgemeinen ist ein Anteil von etwa 3-40 Gew.-%, insbesondere von etwa 5-20 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 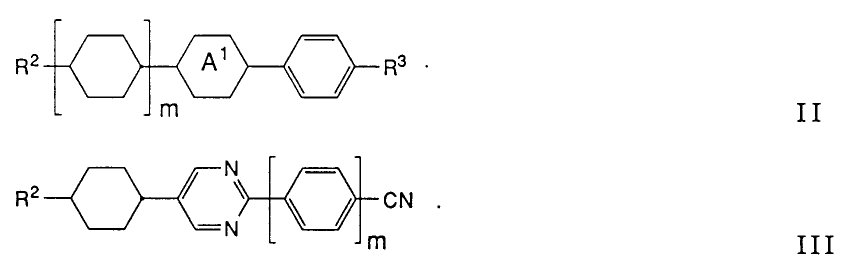 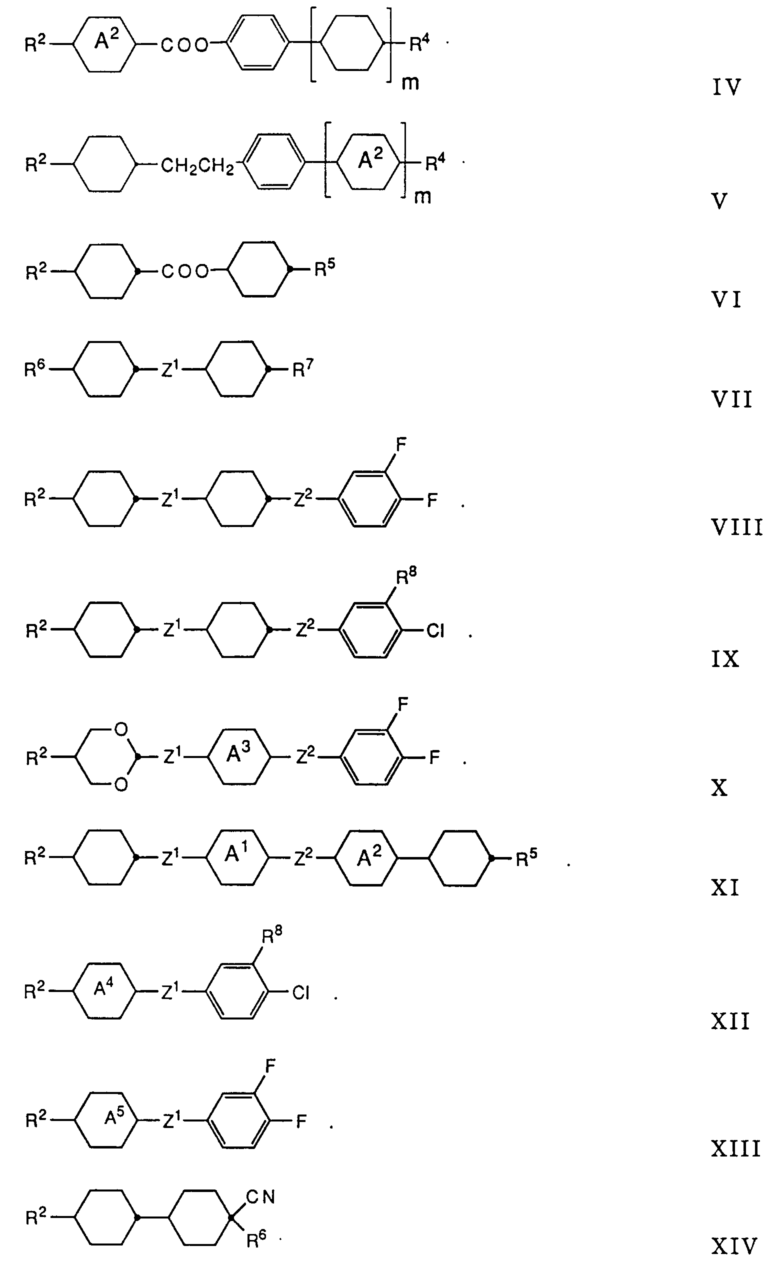 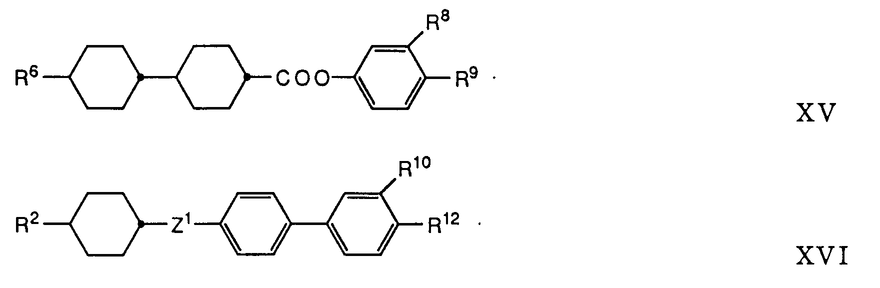 worin
- R²,R⁵: Alkyl, Alkoxyalkyl, 3E-Alkenyl, 4-Alkenyl oder an gesättigten Ringen auch 1E-Alkenyl bedeuten;
- m: 0 oder 1 bedeutet;
- Ring A¹: 1,4-Phenylen, Pyridin-2,5-diyl, Pyrimidin-2,5-diyl, trans-1,4-Cyclohexylen oder trans-1,3-Dioxan-2,5-diyl bedeutet;
- R³: Cyano, Isothiocyanato, Fluor, Alkyl, 3E-Alkenyl, 4-Alkenyl, Alkoxy, 2E-Alkenyloxy, 3-Alkenyloxy oder 1-Alkinyl bedeutet;
- Ring A²: 1,4-Phenylen oder trans-1,4-Cyclohexylen bedeutet;
- Ring A³: 1,4-Phenylen, trans-1,4-Cyclohexylen oder Pyridin-2,5-diyl bedeutet;
- Ring A⁴: trans-1,4-Cyclohexylen oder trans-1,3-Dioxan-2,5-diyl bedeutet;
- Ring A⁵: trans-1,4-Cyclohexylen, trans-1,3-Dioxan-2,5-diyl oder Pyrimidin-2,5-diyl bedeutet;
- R⁴: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bedeutet;
- R⁶: Alkyl, 1E-Alkenyl, 3E-Alkenyl oder 4-Alkenyl bedeutet;
- R⁷: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)-oxymethyl bedeutet;
- Z¹, Z²: eine einfache Kovalenzbindung oder -CH₂CH₂- bedeuten, wobei zwei aromatische Ringe immer durch eine einfache Kovalenzbindung verknüpft sind;
- R⁸: Wasserstoff, Fluor oder Chlor bedeutet;
- R⁹: Cyano, Fluor oder Chlor bedeutet;
- R¹⁰: Wasserstoff oder Fluor bedeutet;
- R¹²: Fluor oder Chlor bedeutet.

Die im Zusammenhang mit den Verbindungen der Formeln II bis XVI verwendeten Ausdrücke werden im folgenden erläutert.
"Aromatische Ringe" bezeichnet Ringe, wie beispielsweise 1,4-Phenylen, Pyridin-2,5-diyl oder Pyrimidin-2,5-diyl.
"Gesättigte Ringe" bezeichnet trans-1,4-Cyclohexylen oder trans-1,3-Dioxan-2,5-diyl.
"Alkyl" bedeutet vorzugsweise geradkettige Gruppen mit 1 bis 12 Kohlenstoffatomen, besonders bevorzugte Reste haben 1 bis 7 Kohlenstoffatomen, wie beispielsweise Methyl, Ethyl, Propyl, Butyl, Pentyl, Hexyl oder Heptyl.
"Alkyloxyalkyl" bedeutet vorzugsweise geradkettige Reste, wie beispielsweise Methoxymethyl, Ethoxymethyl, Propyloxymethyl, Butyloxymethyl und dergleichen.
"Alkyloxy" bedeutet vorzugsweise geradkettige Reste, wie beispielsweise Methoxy, Ethoxy, Propyloxy, Butyloxy, Pentyloxy, Hexyloxy, Heptyloxy und dergleichen.
"1E-Alkenyl" bedeutet vorzugsweise geradkettige Alkenylreste, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.
"3E-Alkenyl" bedeutet vorzugsweise geradkettige Alkenylreste, in denen die Doppelbindung sich in 3-Stellung befindet, wie beispielsweise 3-Butenyl, 3E-Pentenyl, 3E-Hexenyl, 3E-Heptenyl und dergleichen.
"4-Alkenyl" bedeutet vorzugsweise geradkettige Alkenylreste, in denen die Doppelbindung sich in 4-Stellung befindet, wie beispielsweise 4-Pentenyl, 4-Hexenyl, 4-Heptenyl und dergleichen.
"2E- bzw. 3-Alkenyloxy" bedeutet in diesem Zusammenhang vorzugsweise geradkettige Alkenyloxyreste, in denen die Doppelbindung sich in 2- bzw 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.
"1-Alkinyl" bedeutet in diesem Zusammenhang vorzugsweise geradkettige Alkinylreste, in denen die Dreifachbindung sich in 1-Stellung befindet, wie beispielsweise Ethinyl, 1-Propinyl, 1-Butinyl, 1-Pentinyl und dergleichen.

Die erfindungsgemässen Mischungen können ferner optisch aktive Verbindungen (z.B. optisch aktive 4'-Alkyl. oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Anzeigevorrichtungen können in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn bezeichnet die optische Anisotropie.

### Beispiel 1

Zu einer Lösung von 1,2 g 4-Cyano-3-fluorphenol, 1,2 g 4-[(E)-But-2-enoyloxy]benzosäure und 0,1 g 4-(Dimethylamino)pyridin in 50 ml Dichlormethan wurde unter Rühren innert 5 Minuten 2,6 g N,N '-Dicyclohexylcarbodiimid gegeben. Das Reaktionsgemisch wurde über Nacht weitergerührt, dann filtriert. Das Filtrat wurde mit gesättigter Natriumbicarbonat-Lösung und mit Wasser gewaschen und eingeengt. Chromatographische Reinigung des Rückstandes an Kieselgel mit Hexan/Ethylacetat (Vol. 9:1) und zweimaliges Umkristallisieren der gemäss Dünnschichtchromatographie reinen Fraktionen ergab 2,0 g 4-Cyano-3-fluorphenyl 4-[(E)-but-2-enoyloxy]benzoat. Smp. (C-N) 125°C, und Klp. (N-I) 195°C.

Die als Ausgangsmaterial verwendete 4-[(E)-But-2-enoyloxy]benzosäure wurde wie folgt hergestellt:
a) Zu einer Lösung von 7,0 g 4-Hydroxybenzaldehyd, 7,4 g (E)-But-2-ensäure und 0,1 g 4-(Dimethylamino)pyridin in 50 ml Dichlormethan wurde unter Rühren innert 5 Minuten 17,8 g N,N '-Dicyclohexylcarbodiimid gegeben. Das Reaktionsgemisch wurde über Nacht weitergerührt, dann filtriert. Das Filtrat wurde mit gesättigter Natriumbicarbonat-Lösung und mit Wasser gewaschen und eingeengt. Chromatographische Reinigung des Rückstandes an Kieselgel mit Hexan/Ethylacetat (Vol. 7:3) und zweimaliges Umkristallisieren der gemäss Dünnschichtchromatographie reinen Fraktionen ergab 12,2 g 4-[(E)-But-2-enoyloxy]benzaldehyd.
b) Zu einer Lösung von 12,2 g 4-[(E)-But-2-enoyloxy]benzaldehyd und 100 ml Aceton bei 0 °C wurde unter Rühren innert 15 Minuten 50 ml Jones' Reagenz zugetropft. Das Reaktionsgemisch wird über Nacht bei Raumtemperatur weitergerührt, auf Wasser gegossen und dreimal mit je 50 ml Ethylacetat extrahiert. Die vereinigten organischen Phasen wurden zweimal mit je 50 ml 2N Natronlauge extrahiert. Die vereinigten Natronlauge Phasen wurden mit Salzsäure angesäuert (pH 1), dann dreimal mit je 50 ml Ethylacetat extrahiert. Die organischen Phasen wurden vereinigt und zweimal mit je 50 ml Wasser gewaschen, über Magnesiumsulfat getrocknet, filtriert und anschliessend eingeengt. Umkristallisieren des Rückstandes aus Ethanol ergab 10,5 g 4-[(E)-But-2-enoyloxy]benzosäure.

Auf analoge Weise können folgende Verbindungen hergestellt werden:
4-Cyano-3-fluorphenyl 4-[(E)-pent-2-enoyloxy]benzoat, Smp. (C-N) 84°C, Klp. (N-I) 120°C
4-Cyano-3-fluorphenyl 4-[(E)-hex-2-enoyloxy]benzoat, Smp. (C-N) 72°C, Klp. (N-I) 119°C
4-Cyano-3-fluorphenyl 4-[(E)-hept-2-enoyloxy]benzoat, Smp. (C-N) 68°C, Klp. (N-I) 109°C
4-Cyano-3-fluorphenyl 4-[(E)-oct-2-enoyloxy]benzoat, Smp. (C-N) 60°C, Klp. (N-I) 110°C
4-Cyano-3-fluorphenyl 4-[(E)-non-2-enoyloxy]benzoat
4-Cyano-3-fluorphenyl 4-[(E)-dec-2-enoyloxy]benzoat
4-Cyano-3-fluorphenyl 4-[(E)-undec-2-enoyloxy]benzoat
4-Cyano-3-fluorphenyl 4-[(E)-dodec-2-enoyloxy]benzoat
4-Chlor-3-fluorphenyl 4-[(E)-but-2-enoyloxy]benzoat
4-Chlor-3-fluorphenyl 4-[(E)-pent-2-enoyloxy]benzoat
4-Chlor-3-fluorphenyl 4-[(E)-hex-2-enoyloxy]benzoat
4-Chlor-3-fluorphenyl 4-[(E)-hept-2-enoyloxy]benzoat
4-Chlor-3-fluorphenyl 4-[(E)-oct-2-enoyloxy]benzoat
4-Chlor-3-fluorphenyl 4-[(E)-non-2-enoyloxy]benzoat
4-Chlor-3-fluorphenyl 4-[(E)-dec-2-enoyloxy]benzoat
4-Chlor-3-fluorphenyl 4-[(E)-undec-2-enoyloxy]benzoat
4-Chlor-3-fluorphenyl 4-[(E)-dodec-2-enoyloxy]benzoat
3, 4-Difluorphenyl 4-[(E)-but-2-enoyloxy]benzoat
3, 4-Difluorphenyl 4-[(E)-pent-2-enoyloxy]benzoat
3, 4-Difluorphenyl 4-[(E)-hex-2-enoyloxy]benzoat
3, 4-Difluorphenyl 4-[(E)-hept-2-enoyloxy]benzoat
3, 4-Difluorphenyl 4-[(E)-oct-2-enoyloxy]benzoat
3, 4-Difluorphenyl 4-[(E)-non-2-enoyloxy]benzoat
3, 4-Difluorphenyl 4-[(E)-dec-2-enoyloxy]benzoat
3, 4-Difluorphenyl 4-[(E)-undec-2-enoyloxy]benzoat
3, 4-Difluorphenyl 4-[(E)-dodec-2-enoyloxy]benzoat
3, 4, 5-Trifluorphenyl 4-[(E)-but-2-enoyloxy]benzoat
3, 4, 5-Trifluorphenyl 4-[(E)-pent-2-enoyloxy]benzoat
3, 4, 5-Trifluorphenyl 4-[(E)-hex-2-enoyloxy]benzoat
3, 4, 5-Trifluorphenyl 4-[(E)-hept-2-enoyloxy]benzoat
3, 4, 5-Trifluorphenyl 4-[(E)-oct-2-enoyloxy]benzoat
3, 4, 5-Trifluorphenyl 4-[(E)-non-2-enoyloxy]benzoat
3, 4, 5-Trifluorphenyl 4-[(E)-dec-2-enoyloxy]benzoat
3, 4, 5-Trifluorphenyl 4-[(E)-undec-2-enoyloxy]benzoat
3, 4, 5-Trifluorphenyl 4-[(E)-dodec-2-enoyloxy]benzoat

### Beispiel 2

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung wurde in einer TN-Zelle (low bias tilt) mit 8 mm Plattenabstand bei 22°C gemessen; als Betriebsspannung wurde der 2,5-fache Wert der Schwellenspannung (V₁₀) gewählt. Gemessen wurden der Klärpunkt [Klp. (N-I)], die Schwellenspannung (V₁₀), die Einschaltzeit (tₒₙ), die Ausschaltzeit (t_{off}) sowie die optische Anisotropie (Δn). Die entsprechenden Daten für 4-(trans-4-Pentylcyclohexyl)benzonitril betragen: Klp. (N-I) 54,6°C, V₁₀ = 1,62 V, tₒₙ = 22 ms, t_{off} = 40 ms und Δn = 0,120.

### BM-1

- 90 Gew.%: 4-(trans-4-Pentylcyclohexyl)benzonitril
- 10 Gew.%: 4-Cyano-3-fluorphenyl 4-[(E)-but-2-enoyloxy]benzoat.
Klp. (N-I) = 61,5 °C, V₁₀ = 1,37 V, Tₒₙ = 31 ms, T_{off} = 51 ms, Δn = 0,129.

### BM-2

- 80 Gew.%: 4-(trans-4-Pentylcyclohexyl)benzonitril
- 20 Gew.%: 4-Cyano-3-fluorphenyl 4-[(E)-but-2-enoyloxy]benzoat.
Klp. (N-I) = 69,6 °C, V₁₀ = 1,20 V, Tₒₙ = 43 ms, T_{off} = 70 ms, Δn = 0,136.

## Patentansprüche

1. Verbindungen der allgemeinen Formel 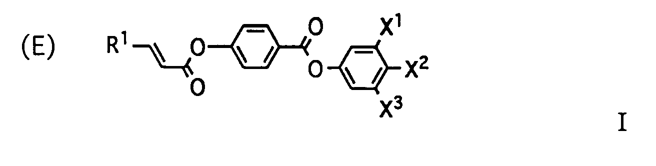 worin
R¹ Alkyl mit 1 bis 12 Kohlenstoffatomen;
X¹, X³ unabhängig voneinander Halogen oder Wasserstoff; und
X² Halogen oder Cyano bezeichnen.

2. Verbindungen gemäss Anspruch 1, dadurch gekennzeichnet, dass X¹ Fluor, X² Chlor, Fluor oder Cyano und X³ Wasserstoff oder Fluor bedeutet.

3. Verbindungen gemäss einem der Ansprüche 1 oder 2 der allgemeinen Formeln 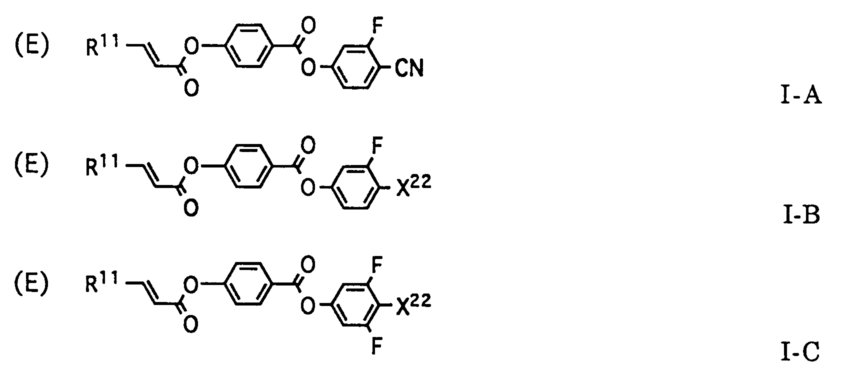 worin
R¹¹ Alkyl mit 1 bis 7 Kohlenstoffatomen; und
X²² Fluor oder Chlor darstellt.

4. Verbindung gemäss einem der Ansprüche 1 bis 3, dadurch gekennzeichnet, dass R¹ ein Alkylrest mit 1 bis 3 Kohlenstoffatomen bedeutet.

5. Die Verbindung der Formel

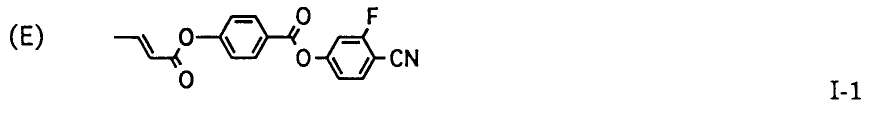

6. Verbindungen der Formeln 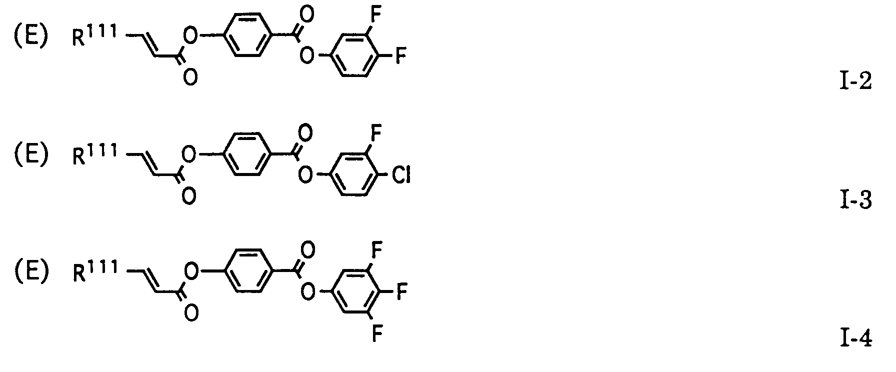 worin R¹¹¹ Alkyl mit 1 bis 3 Kohlenstoffatomen bedeutet.

7. Flüssigkristallines Gemisch mit mindestens 2 Komponenten, dadurch gekennzeichnet, dass mindestens eine Komponente eine Verbindung der in Anspruch 1 definierten Formel I ist.

8. Verwendung der Verbindungen der in Anspruch 1 definierten Formel I für elektro-optische Vorrichtungen.

## Claims

1. Compounds of the general formula 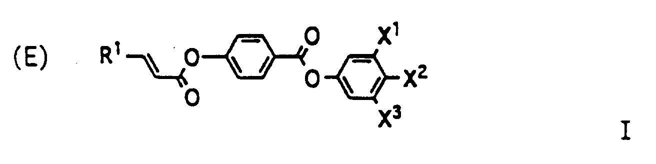 in which
R¹ is alkyl having 1 to 12 carbon atoms;
X¹ and X³, independently of one another, are halogen or hydrogen;
X² is halogen or cyano.

2. Compounds according to Claim 1, characterized in that X¹ is fluorine, X² is chlorine, fluorine or cyano and X³ is hydrogen or fluorine.

3. Compounds according to one of Claims 1 or 2, of the general formulae 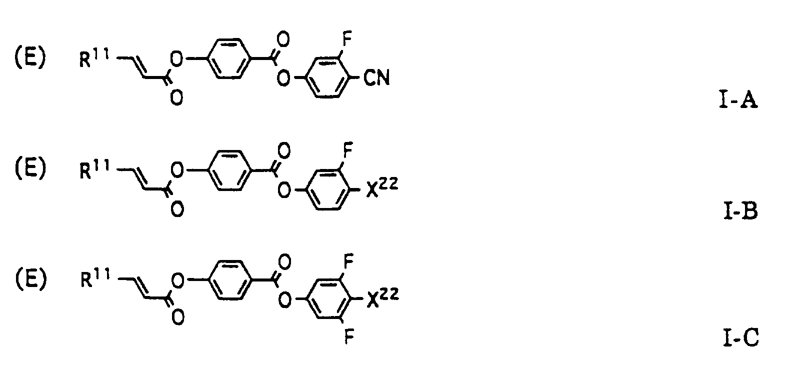 in which
R¹¹ is alkyl having 1 to 7 carbon atoms; and
X²² is fluorine or chlorine.

4. Compound according to one of Claims 1 to 3, characterized in that R¹ is an alkyl radical having 1 to 3 carbon atoms.

5. The compound of the formula

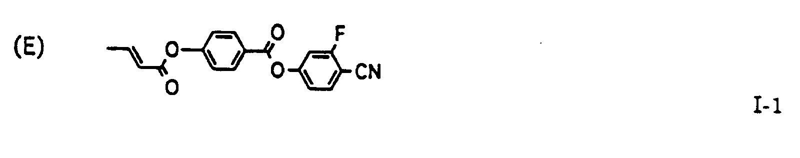

6. Compounds of the formulae 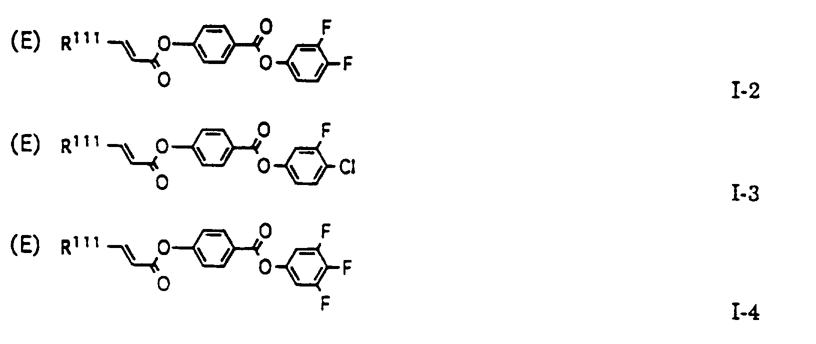 in which R¹¹¹ is alkyl having 1 to 3 carbon atoms.

7. Liquid-crystalline mixture having at least 2 components, characterized in that at least one component is a compound of the formula I defined in Claim 1.

8. Use of the compounds of the formula I defined in Claim 1 for electro-optical devices.

## Revendications

1. Composés de formule générale 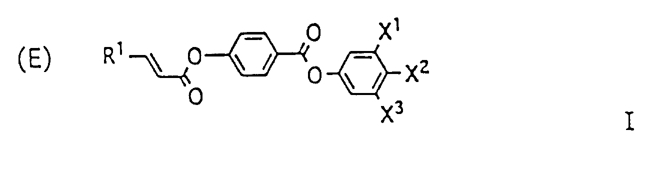 dans laquelle
R¹ représente un groupe alkyle en C₁-C₁₂ ;
X¹ et X³ représentent chacun, indépendamment l'un de l'autre, un halogène ou l'hydrogène ; et
X² représente un halogène ou le groupe cyano.

2. Composés selon revendication 1, caractérisés en ce que X¹ représente le fluor, X² le chlore, le fluor ou un groupe cyano et X³ l'hydrogène ou le fluor.

3. Composés selon l'une des revendications 1 ou 2, répondant aux formules générales 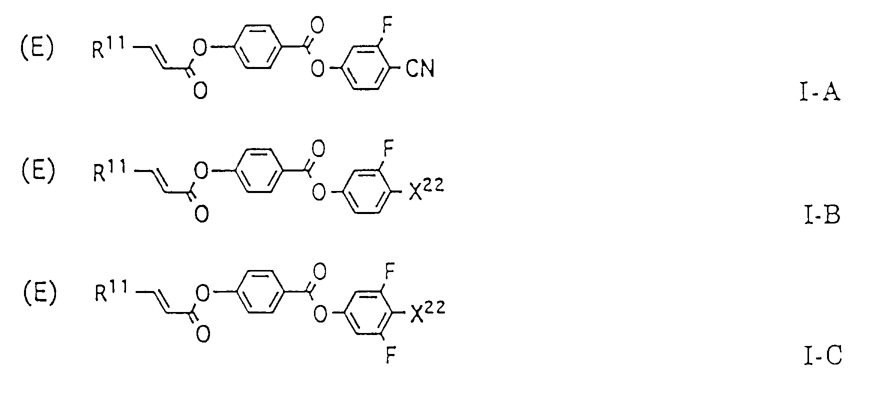 dans lesquelles
R¹¹ représente un groupe alkyle en C₁-C₇ et
X²² représente le fluor ou le chlore.

4. Composés selon l'une des revendications 1 à 3, caractérisé en ce que R¹ représente un groupe alkyle en C₁-C₃.

5. Le composé de formule

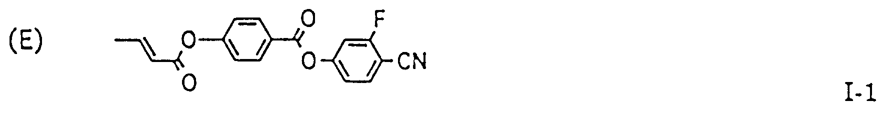

6. Composés de formules 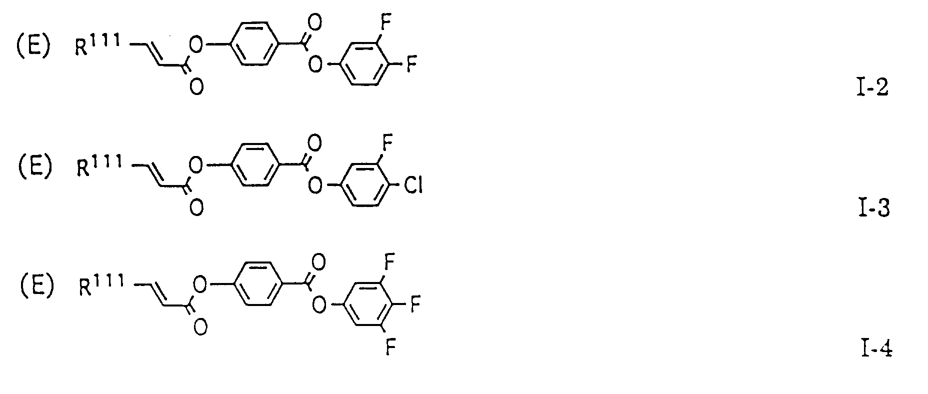 dans lesquelles R¹¹¹ représente un groupe alkyle en C₁-C₃.

7. Mélange à cristaux liquides à au moins deux composants, caractérisé en ce que l'un au moins des composants est un composé de formule I de la revendication 1.

8. Utilisation des composés de formule I de la revendication 1 dans des dispositifs électro-optiques.
